Europäisches Patentamt

European Patent Office

Office européen des brevets

(11) Publication number : **0 650 720 A1**

# (12) EUROPEAN PATENT APPLICATION

(21) Application number : **94307020.1**

(22) Date of filing : **26.09.94**

(51) Int. Cl.[6] : **A61K 7/32,** A61K 7/48, A61K 31/66

(30) Priority : **28.10.93 JP 270704/93**
**28.12.93 JP 335230/93**

(43) Date of publication of application :
**03.05.95 Bulletin 95/18**

(84) Designated Contracting States :
**CH DE FR GB IT LI**

(71) Applicant : **SANWA KAGAKU KENKYUSHO CO., LTD.**
**No. 35, Higashi-sotobori-cho**
**Higashi-ku Nagoya-shi Aichi-ken (JP)**

(72) Inventor : **Sawai, Kiichi**
**2-19-9, 2ban, Atuta-ku,**
**Nagoya-shi**
**Aichi-ken (JP)**
Inventor : **Mitani, Takahiko**
**15-1, Senjigureshinden, Hokusei-cho**
**Inabe-gun Mie-ken (JP)**
Inventor : **Ninomiya, Naohisa**
**20-66, Aza Minamitujinouchi,**
**Nakashima,**
**Kitakata-cho, Ichinomiya Aichi-ken (JP)**
Inventor : **Tsurumi, Yoshimi**
**342, Neba, Oaza Gomei-cho, Konan-shi**
**Aichi-ken (JP)**

(74) Representative : **Bubb, Antony John Allen et al**
**GEE & CO.**
**Chancery House**
**Chancery Lane**
**London WC2A 1QU (GB)**

(54) **Skin care and deodorant compositions.**

(57)  A skin care or deodorant composition comprises phytic acid in the form of a salt with at least one metal ion preferably selected from sodium, potassium, calcium, and magnesium ions.

**EP 0 650 720 A1**

The present invention relates generally to a composition in which phytic acid is present in the form of a salt with a metal ion, and more particularly to a skin care or deodorant composition which comprises a phytic acid metal salt.

In the present disclosure, the term "skin care composition" is understood to include skin beautifiers, facial creams and other preparations having some considerable action on ameliorating corneous tissue and preventing the aging of the skin, and the term "deodorant composition" is understood to refer to various forms of compositions that can be used to remove bad or foul smells from every site on the body of humans or warm blooded animals.

Phytic acid has long been known as a chelating agent, and used in the form of additives for foods and industrial products. However, the inventors have recently found that phytic acid, when administrated perorally to humans, has actions not only on removing uraroma and other offensive smells but also on medicating metabolic diseases (see JP-B 4-76971, JP-B 5-39929, and USP 4,929,438).

White cosmetics making use of the action of kojic acid, etc, on inhibiting tyrosinase are also known (JP-A 63-208510).

Skin beautifiers are now studied from physical aspects of inhibiting skin secretions, removing foul smells from such secretions and providing protection against the skin, as well as from their destructive aspects, and used to this end are primarily cosmetics and deodorants composed of alcohols, oils and fats, and perfumes or aromatics. However, almost all of these provide only aesthetic effect. Acne, and moles and warts parasitic on the skin, are known to manifest themselves due to metabolic dysfunction, pathogenic microparasites, and tissue dysfunction caused by exposure to light and to toxic substances. These disorders are treated merely by surgical removal of disordered tissue (eg, cauterization with salicylate drugs, and freezing of cells with liquid nitrogen), removal of pathogenic substances, and cleansing of the skin; and never until now has any efficacious procedure of preventing and medicating these disorders been established.

Body smell is derived from sweat secreting from sweat glands such as eccrine and apocrine glands, but this sweat itself is odorless. In the mechanism of generating body smell, it is believed that sweat from eccrine and apocrine glands, keratin protein derived from epidermic cell, etc, cause odoriferous substances such as ammonia, lower fatty acids, aldehyde, and alcohol to be produced under the action of bacterial protease and urease from cutaneous cells; or cause lipid to be converted into fatty acids under the action of bacterial lipase, and such fatty acids to be further oxidized to produce odoriferous substances such as lower fatty acids and $\tau$-lactone, resulting in body smell.

Aluminum compound drugs or anhidrotic drugs for causing cutaneous proteins to close up sweat glands and thereby prevent exposure of sweat to the skin, and deodorizing components such as bactericides, antioxidants and aromatics, are generally used as drugs expected to have beneficial or aesthetic action on the skin and a deodorizing action by removal of such body smell. However, they have strong toxicity to the skin, and so must be used at low doses, thus failing to provide any sufficient effect on the skin.

Toilet soaps, etc, are effective to keep the skin clean for a short period of time. Never until now, however, has there been any report on keeping the skin clean for a prolonged period of time with the use of such soaps.

More specifically, the present invention provides an activated phytic acid composition in which phytic acid forms a salt with at least one metal ion.

Preferably, the metal ion is selected from the group of consisting of sodium, potassium, calcium, and magnesium ions.

Preferably, the composition contains up to 50% (w/v) of phytic acid and is used as a skin care composition or as a deodorant composition.

As a result of intensive studies, the inventors have now found that phytic acid has some remarkable actions on ameliorating corneous tissue and preventing the aging of the skin; and an activated phytic acid composition in which phytic acid forms a salt with at least one metal ion has further improved actions on skin care without irritating the skin.

Pimples are a form of inflammation with a foul smell. Secretions such as sweat are decomposed into fatty acids, etc., by bacteria on the surface of the skin. These fatty acids are then decomposed through oxidation into odoriferous substances such as lower fatty acids, which in turn destroy surrounding cells.

Moles and warts are induced by the variation of normal cells by exposure to the rays of the sun, toxic substances, and secretions from viruses and bacteria proliferated by secretions such as sweat on the surface of the skin.

Although not fully understood, it is believed that the activated phytic acid composition containing a phytic acid salt according to the present invention acts as an enzyme inhibitor for urease, lipase, protease and other enzymes produced by viruses and bacteria, and as an antioxidant as well, thereby having an excellent aesthetic action on the skin.

Although not fully understood, it is believed that the reason the activated phytic acid composition of the invention behaves as an enzyme inhibitor for urease, lipase, and protease is that it acts effectively as a chelating agent to deprive bacterial enzymes of trace metals and thereby deactivate the enzymes. It is also believed that the reason the present composition behaves as an antioxidant is that it acts effectively as a chelating agent to sequestrate trace metals that are a catalyst for oxidation.

The salt of phytic acid with at least one metal ion has a moderate action on the living body, but does not irritate the skin. Although not fully understood, it is believed that the salt of phytic acid with a metal (e.g., Na, K, Ca or Mg) capable of being substituted by the trace metal required for bacterial enzymes can moderate the intense chelating action of phytic acid itself, so that it can have a moderate action on the living body and, at the same time, can act as an enzyme inhibitor. Lack of irritation to the skin is of great importance, because the deodorant composition is used over a wide area for a prolonged period of time.

Rice germ oil itself is known to have a great aesthetic action on the skin. However, such an action is often not obtained, because rice germ oil is unusually unstable due to oxidation.

The activated phytic acid composition of the invention enables rice germ oil to have an enhanced aesthetic action on the skin. Although not fully understood, it is believed that rice germ oil cannot only increase the stability of the composition during storage but can also maintain the stability and activity of the composition on site.

The activated phytic acid composition of the invention should preferably contain up to 50% (w/v) of phytic acid. The metal that forms a salt with phytic acid should preferably be Na, K, Ca or Mg. It is then preferable that phytic acid is used with a monovalent metal at a molar ratio of 1:6 to 1:12, and with a divalent metal at a molar ratio of 1:3 to 1:6.

The present invention will now be explained more illustratively by reference to Preparation Examples and Experiments.

## PREPARATION EXAMPLES

### Prep. Ex. 1 - Shampoo

A suitable amount of water was added to phytic acid and magnesium hydroxide at a molar ratio of 1:6 to prepare an aqueous solution containing 5% of phytic acid (activated phytic acid composition A), which was then used to prepare a shampoo according to the following recipe in a conventional manner.

| | |
|---|---|
| Activated phytic acid composition A | 2 ml |
| Alkyl ether sulfuric acid sodium salt | 16 g |
| Diethanolamide laurate | 4 g |
| Propylene glycol | 2 g |
| Water | suitable |
| Total | 100 ml |

### Prep. Ex. 2 - Shampoo

A suitable amount of water was added to phytic acid and potassium hydroxide at a molar ratio of 1:6 to prepare an aqueous solution containing 20% of phytic acid (activated phytic acid composition B), which was then used to prepare a shampoo according to the following recipe in a conventional manner.

| | |
|---|---|
| Activated phytic acid composition B | 25 ml |
| Amisoft CT-12 | 15 g |
| (Coconut oil fatty acid cetyl-L-glutamic acid monoethanolamine 30%) | |
| Sodium lauryl sulfate ether | 16 g |
| Coconut oil fatty acid diethanolamide | 4.5 g |
| PCA soda (50%) | 1 g |
| Water | suitable |
| Total | 100 ml |

Prep. Ex. 3 - Additive for bath water

A suitable amount of water was added to phytic acid and potassium hydroxide at a molar ratio of 1:12 to prepare an aqueous solution containing 20% of phytic acid (activated phytic acid composition C),which was then used to prepare bath water additive according to the following recipe in a conventional manner.

| | |
|---|---|
| Activated phytic acid composition C | 20 ml |
| Light liquid paraffin | 40 g |
| Isopropyl palmitate | 10 g |
| Sorbitan monostearate | 3.5 g |
| Polyoxyethylene stearyl ether | 4.5 g |
| Water | suitable |
| Total | 100 ml |

Prep. Ex. 4 - Aerosol

A suitable amount of water was added to phytic acid and sodium hydroxide at a molar ratio of 1:8 to prepare an aqueous solution containing 50% of phytic acid (activated phytic acid composition D),which was then packed in an injector to prepare an aerosol in a conventional manner.

Prep. Ex. 5 - Cream

A suitable amount of water was added to phytic acid and sodium hydroxide at a molar ratio of 1:8 to prepare an aqueous solution containing 5% of phytic acid (activated phytic acid composition E), which was then used to prepare cream according to the following recipe in a conventional manner.

| Activated phytic acid composition E | 10 ml |
|---|---|
| Rice germ oil | 1 g |
| Potassium stearate | 7 g |
| Stearic acid | 15 g |
| Glycerin | 5 g |
| Water | suitable |
| Total | 100 g |

Prep. Ex. 6 - Facial cream

The activated phytic acid composition B according to Prep. Ex. 2 was used to prepare a facial cream according to the following recipe in a conventional manner.

| Activated phytic acid composition B | 20 ml |
|---|---|
| Rice germ oil | 0.5 g |
| Amisoft CT-11 | 15 g |
| (N-lauroyl-L-glutamic acid monosodium salt 100%) | |
| Diethanolamide laurate | 5 g |
| Polyethylene glycol (6,300) monostearate | 5 g |
| Propylene glycol | 10 g |
| PCA soda (50%) | 5 g |
| Water | suitable |
| Total | 100 g |

Prep. Ex. 7 - Oral Deodorant

A suitable amount of water was added to phytic acid and calcium hydroxide at a molar ratio of 1:3 to prepare an aqueous solution containing 5% of phytic acid or an activated phytic acid composition F, which was then used to prepare an oral deodorant according to the following recipe in a conventional manner.

| Activated phytic acid composition F | 10 ml |
|---|---|
| Ethanol | 8 ml |
| Sodium fluoride | 0.05 g |
| Water | suitable |
| Total | 100 ml |

Comp. Ex. 1 - Shampoo

A shampoo was prepared according to the following recipe in a conventional manner.

5

| 50% phytic acid aqueous solution | 0.2 ml |
|---|---|
| Alkyl ether sulfuric acid sodium salt | 16 g |
| Diethanolamide laurate | 4 g |
| Propylene glycol | 2 g |
| Water | suitable |
| Total | 100 ml |

Comp. Ex. 2 - Cream

Cream was prepared according to the following recipe in a conventional manner.

| Potassium stearate | 7 g |
|---|---|
| Stearic acid | 15 g |
| Glycerin | 5 g |
| Water | suitable |
| Total | 100 g |

Comp. Ex. 3 - Cream

Cream was prepared according to the following recipe in a conventional manner.

| Rice germ oil | 1 g |
|---|---|
| Potassium stearate | 7 g |
| Stearic acid | 15 g |
| Glycerin | 5 g |
| Water | suitable |
| Total | 100 g |

## EXPERIMENTS

## ACTION ON AMELIORATING CORNEOUS TISSUE AND PREVENTING THE AGING OF THE SKIN

## Experiment Protocol

Nude mice of five to seven week ages were irradiated with ultraviolet rays at a UVB dose of 0.05 $J/cm^2$ over five months. The total dose was 4 $J/cm^2$.

After irradiated with ultraviolet rays, the cream (100 to 1,000 ug) according to Prep. Ex. 1 was applied on a 10-$cm^2$ area of the back of each mouse three times a week over a period of ten weeks. After this, the mouse was euthanized in a humane manner for skin isolation. That creamed area was hollowed out in a disk form, and the disk was coated with silicone (SILFLO), followed by curing. The silicone film was removed from the skin disk to form a skin model. This model was then irradiated with light at a certain low angle to make estimation of the widths of shadows produced thereon by wrinkles and asperities on the basis of the wrinkle index of 0 to 4. Index 0 shows that no wrinkles were observed at all, whereas Index 4 shows that the widths of shadows reached a maximum.

## Results

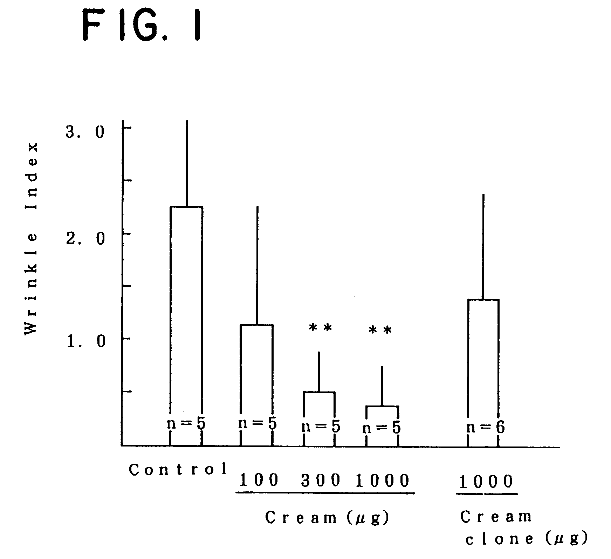

As can be seen from FIG. 1, it was observed that the application of the present cream gave rise to a significant decrease in the wrinkle index, indicating that the present cream has a demonstrable anesthetic action.

## ACTION ON INHIBITING BODY SMELL AND EARWAX

## Experiment Protocol

(A) Three intact beagles weighing about 20 kg, male and two-year old, were used. The animals were washed with the shampoos according to Prep. Examples. 1 and 2 and Comp. Ex. 1. Several days later the animals were estimated in terms of body smell.

(B) Both the ears of the animals were cleaned by use of applicators and alcoholized cotton. One ears were fully treated with (1) the cream according to Prep. Ex. 5, (2) an agent containing 500 mg of phytic acid per 100 g of the cream according to Comp. Ex. 2, and (3) the cream according to Comp. Ex. 2, whereas the other ears were not.

Three days after the treatment, the animals were estimated in terms of body smell and earwax. That is, the earwax removed by an earpick was weighed, and the body smell was evaluated by having experts smell the manes.

## Results

As can be seen from the results set out below, it is found that the shampoos according to Prep. Examples 1 and 2 have a demonstrable action on inhibiting mane smell, whereas the cream according to Prep. Ex. 5 has a demonstrable action of inhibiting earwax and ear smell. However, the creams according to Comp. Examples 2 and 3 had no action at all.

|  | Earwax, g | | Smell | | |
|  |  |  | Ears | | Mane |
|  | (T) | (U) | (T) | (U) |  |
| (A) Shampoo of Prep. Ex. 1 |  |  |  |  | -1 |
| (B) Cream of Prep. Ex. 5 | 0.05 | 0.7 | -1 | -4 |  |
| (A) Shampoo of Prep. Ex. 2 |  |  |  |  | -2 |
| (B) Cream of the above (2) | 0.34 | 0.72 | -3 | -4 |  |
| (A) Shampoo of Comp. Ex. 1 |  |  |  |  | -4 |
| (B) Cream of Comp. Ex. 2 | 0.74 | 0.71 | -4 | -4 |  |
| (T): Treated |  |  |  |  |  |
| (U): Untreated |  |  |  |  |  |

The deodorizing effect was evaluated by experts on the following five ranks.

| Rank | Smell |
|---|---|
| 0 | Not offensive |
| -1 | Slightly offensive |
| -2 | Offensive |
| -3 | Very offensive |
| -4 | Extremely offensive |

It is here noted that the ranks "0" and "-1" are acceptable.

EVALUATION OF DEODORIZING EFFECT AND SKIN IRRITATION

Experiment Protocol

The deodorizing effect was evaluated as mentioned above. Skin irritation was evaluated by having subjects reply to a questionnaire.

Skin irritation was evaluated by five male subjects having strong body smell or foul breath with relatively sensitive skin irritations. The deodorizing effect was evaluated prior to experimentation and at the third and seventh days after the onset of experimentation, whereas skin irritation was evaluated at the seventh day.

Results

As can be seen from the results set out below, the inventive products have a demonstrable deodorizing effect without giving irritation on the skin.

| Subject | PUT | STE | Deodorizing Effect (A) | (B) | (C) | Irritation |
|---|---|---|---|---|---|---|
| a | CP | Body smell | -4 | -3 | -3 | Slightly strong |
| a | CE1 | Body smell | -3 | -2 | -2 | Strong |
| a | PE1 | Body smell | -4 | -1 | -0 | No irritation |
| b | CP | Body smell | -3 | -3 | -3 | Strong |
| b | CE1 | Body smell | -3 | -3 | -2 | No irritation |
| b | PE2 | Body smell | -4 | -0 | -0 | No irritation |
| c | PE7 | Foul breath | -4 | -1 | -1 | No irritation |
| d | PE3 | Body smell | -4 | -1 | -0 | No irritation |
| e | PE4 | Body small | -4 | -0 | -0 | No irritation |

PUE: Product under experimentation

STE: Smell to be evaluated

(A): Prior to experimentation

(B): Third day

(C): Seventh day

CP: Commercial product containing bactericides

CE1: Comp. Ex. 1

PE1: Prep. Ex. 1

PE3: Prep. Ex. 3

PE4: Prep. Ex. 4

PE7: Prep. Ex. 7

ANESTHETIC ACTION ON THE SKIN

Experiment Protocol

The creams according to Prep. Ex. 5 (containing 0.5% of phytic acid and 1% of rice germ oil), Comp. Ex. 2 (containing neither phytic acid nor rice germ oil), and Comp. Ex. 3 (containing 1% of rice germ oil with no phytate) were estimated by having three female volunteers use each cream for a period of seven days and asking them about the anesthetic effect. The inventive cream was found to have a demonstrable anesthetic effect on the skin.

Results

| Volunteer | Cream | Chapping |
|:---:|---|---|
| f | Comp. Ex. 2 | Remaining unchanged or chapped |
| f | Comp. Ex. 3 | Remaining unchanged or chapped |
| f | Prep. Ex. 5 | Demonstrably ameliorated |
| g | Comp. Ex. 2 | Remaining unchanged or chapped |
| g | Comp. Ex. 3 | Remaining unchanged or chapped |
| g | Prep. Ex. 5 | Demonstrably ameliorated |
| h | Comp. Ex. 2 | Remaining unchanged or chapped |
| h | Comp. Ex. 3 | Remaining unchanged or chapped |
| h | Prep. Ex. 5 | Demonstrably ameliorated |

ACTION ON AMELIORATING ACNE

Experiment Protocol

The agent for bath according to Prep. Ex. 3, the cream according to Prep. Ex. 5 and the facial cream according to Prep. Ex. 6 were estimated by having ten pimpled volunteers use them for a certain period of time and, then, response to a questionnaire.

Results

Most of them reported that acne was demonstrably ameliorated with some consideration removal of pigments from moles.

Reply to Questionnaire with regard to Agent for Bath

| | Strong | Good | None |
|---|:---:|:---:|:---:|
| (1) Warm-water effect | 5 | 4 | 1 |
| (2) Refreshing effect after bathing | 4 | 5 | 1 |
| (3) Effect on releasing itching after bathing | 6 | 4 | 0 |
| (4) Effect on inhibiting secretion of sebum after bathing | 3 | 7 | 0 |
| (5) Effect on inhibiting acne | 5 | 5 | 0 |

As can be understood from what has been described above, the present activated phytic acid composition can be used in combination with toilet soaps, etc., not only to keep the skin in clean condition for a prolonged period of time, but also to produce a demonstrable action on removal of body smell without giving irritation on the skin. These effects can be further enhanced by use of rice germ oil.

**Claims**

1. A salt of phytic acid for use in medicine or as a deodorant.

2. Use of a phytic acid salt for the manufacture of a therapeutic composition for the preservation or improvement of the conditions of human or mammalian skin, or use as a deodorant.

3. A salt as claimed in claim 1 or use as claimed in claim 2, wherein the salt is a metal salt.

4. A salt as claimed in claim 1 or 3 or use as claimed in claim 2 or 3, wherein the salt is a sodium, potassium, calcium salt.

5. A skin care or deodorant composition comprising a salt of phytic acid with at least one metal ion.

6. A composition as claimed in claim 5, wherein the metal ion is selected from sodium, potassium, calcium and magnesium ions.

7. A composition as claimed in claim 5 or 6 and which contains up to 50% (W/V) of phytic acid.

8. A composition as claimed in claim 5, 6 or 7 and formulated as a shampoo, bath water additive, aerosol, skin cream, body deodorant or oral deodorant.

# F IG. I

EP 0 650 720 A1

## EUROPEAN SEARCH REPORT

European Patent
Office

**Application Number**

EP 94 30 7020

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | EP-A-0 326 963 (SANWA KAGAKU KENKYUSHO CO.) 9 August 1989 <br> * the whole document * | 1-8 | A61K7/32 <br> A61K7/48 <br> A61K31/66 |
| P,X | EP-A-0 595 690 (DANIEL JOUVANCE) 4 May 1994 <br> * the whole document * | 1-3,5,7,8 | |
| X | EP-A-0 550 279 (UNILEVER PLC.) 7 July 1993 <br> * the whole document * | 1-8 | |
| X | PATENT ABSTRACTS OF JAPAN <br> vol. 11, no. 251 (C-440) <br> & JP-A-62 056 411 (SHISEIDO CO LTD) 12 March 1987 <br> * abstract * | 1-8 | |
| X | DATABASE WPI <br> Week 8124, <br> Derwent Publications Ltd., London, GB; <br> AN 81-43006D <br> & JP-A-56 045 408 (LION DENTIFRICE KK) 25 April 1981 <br> * abstract * | 1-8 | TECHNICAL FIELDS SEARCHED (Int.Cl.6) <br><br> A61K |
| X | DATABASE WPI <br> Week 8122, <br> Derwent Publications Ltd., London, GB; <br> AN 81-39260D <br> & JP-A-56 039 008 (LION DENTIFRICE KK) 14 April 1981 <br> * abstract * | 1-8 | |
| X | PATENT ABSTRACTS OF JAPAN <br> vol. 11, no. 80 (C-409) <br> & JP-A-61 233 612 (KAO CORP) 17 October 1986 <br> * abstract * | 1-8 | |

-/--

The present search report has been drawn up for all claims

| Place of search | Date of completion of the search | Examiner |
|---|---|---|
| THE HAGUE | 21 February 1995 | Couckuyt, P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

**European Patent Office**

## EUROPEAN SEARCH REPORT

Application Number

EP 94 30 7020

### DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | DATABASE WPI<br>Week 8624,<br>Derwent Publications Ltd., London, GB;<br>AN 86-275544<br>& JP-A-61 200 905 (KAO CORP) 5 September 1986<br>* abstract * | 1-8 | |
| X | PATENT ABSTRACTS OF JAPAN<br>vol. 11, no. 85 (C-410)<br>& JP-A-61 238 714 (SHISEIDO CO LTD) 24 October 1986<br>* abstract * | 1,5-8 | |
| X | WO-A-93 07850 (THE PROCTER & GAMBLE COMPANY) 29 April 1993<br>* claims * | 1-3,5,7,8 | |
| P,X | EP-A-0 583 888 (UNILEVER PLC.) 23 February 1994<br>* the whole document * | 1-8 | TECHNICAL FIELDS SEARCHED (Int.Cl.6) |
| X | EP-A-0 332 260 (THE PROCTER & GAMBLE COMPANY) 13 September 1989<br>* the whole document * | 1-8 | |
| X | WO-A-93 11739 (DOW CORNING CORPORATION) 24 June 1993<br>* the whole document * | 1 | |
| X | EP-A-0 409 494 (SANWA KAGAKU KENKYUSHO CO. LTD) 23 January 1991<br>* the whole document * | 1 | |
| X | EP-A-0 390 574 (SANWA KAGAKU KENKYUSHO CO. LTD) 3 October 1990<br>* the whole document * | 1 | |

-/--

The present search report has been drawn up for all claims

| Place of search | Date of completion of the search | Examiner |
|---|---|---|
| THE HAGUE | 21 February 1995 | Couckuyt, P |

**European Patent Office**

## EUROPEAN SEARCH REPORT

Application Number

EP 94 30 7020

| | DOCUMENTS CONSIDERED TO BE RELEVANT | | |
|---|---|---|---|
| Category | Citation of document with indication, where appropriate, of relevant passages | Relevant to claim | CLASSIFICATION OF THE APPLICATION (Int.Cl.6) |
| X | EP-A-0 342 956 (SANWA KAGAKU KENKYUSHO CO. LTD) 23 November 1989 <br> * the whole document * <br> --- | 1 | |
| X | EP-A-0 342 955 (SANWA KAGAKU KENKYUSHO CO. LTD) 23 November 1989 <br> * the whole document * <br> --- | 1 | |
| X | EP-A-0 349 143 (SANWA KAGAKU KENKYUSHO CO. LTD) 3 January 1990 <br> * the whole document * <br> --- | 1 | |
| X | EP-A-0 344 997 (SANWA KAGAKU KENKYUSHO CO. LTD) 6 December 1989 <br> * the whole document * <br> --- | 1 | |
| X | EP-A-0 341 810 (SANWA KAGAKU KENKYUSHO CO. LTD) 15 November 1989 <br> * the whole document * <br> ----- | 1 | |
| | | | TECHNICAL FIELDS SEARCHED (Int.Cl.6) |

The present search report has been drawn up for all claims

| Place of search | Date of completion of the search | Examiner |
|---|---|---|
| THE HAGUE | 21 February 1995 | Couckuyt, P |